# EUROPEAN PATENT APPLICATION

(11) **EP 3 896 241 A1**
(43) Date of publication of application: **20.10.2021**
(21) Application number: 21167944.4
(22) Date of filing: 12.04.2021
(51) Int. Cl.: E05B 1/00, A61L 2/08

(54) **A CABINET HANDLE, AND CABINET INCORPORATING SUCH A HANDLE**

(30) Priority: 16.04.2020 EP 20169988
(71) Applicant: Nualight Limited, Cork (IE)
(72) Inventor: CRONIN, Andrew, West Sussex, RH16 3QX (GB); COLE, Ben, West Sussex, RH10 6LF (GB); NORRIS, Brian, West Sussex, RH16 4GX (GB)
(74) Representative: Weldon O'Brien Ltd.

(57) **Abstract**

A handle (1) for a cabinet, has a translucent rod (2) with a photocatalytic antimicrobial TiO2 external coating (50), and a pair of supports (3, 4) each engaging an end of the rod and being affixed to a cabinet door. One support has an array of two LEDs for emission of light into the rod, one LED emitting blue light and the other emitting white light. There is total internal reflection within the rod, for very effective incidence on the coating.

## Description

### Introduction

The invention relates to handles for use in for example a cabinet such as a refrigerated cabinet.

It is known that there are many advantages to reducing risk of contamination of the hands by contact with a handle. CN208106087 describes an antibiotic aluminium handle, GB2555825 describes an approach in which the handle is covered by a self-cleaning material, CA2889370 describes a handle with an anti-microbial coating, WO2017025705 describes a handle with a thermoplastic polymer material with an embedded anti-microbial agent, US2006006678 describes a handle hand-hold with a foaming agent, and CN2665634 describes a handle with an anti-microbial layer.

The present invention is directed towards providing a handle which is particularly effective at antimicrobial activity and is suitable for a high-usage environment such as in a retail store or any situation where there is frequent human physical contact.

### Summary of the Invention

We describe a handle comprising:
a translucent hand-grip rod with a photocatalytic antimicrobial external coating,
at least one support engaging an end of the rod and comprising a base adapted to be affixed to an apparatus, and
an array of at least one LED for emission of light to activate the coating.

Preferably, the handle comprises at least two said supports, each engaging an opposed end of the rod. Preferably, the at least one support base is adapted to fix to a door. Preferably, the LED array is mounted in at least one said support for emission of light into an end of the rod. Preferably, the rod is configured for total internal reflection. Preferably, the rod has a mirror across an end of the rod opposite to the end through which emitted light enters.

In one example, the rod comprises borosilicate glass material.

Preferably, the coating includes TiO2. Preferably, the TiO2 coating comprises a nano TiO2 polymer, with TiO2 nanoparticles, with particle sizes of average diameter between 1nm and 15nm. Preferably, rod antimicrobial coating has a thickness in the range of 50nm to 120nm.

Preferably, at least one support includes an antimicrobial exposed surface, in one example an alloy surface. In one example, the alloy includes Cu, optionally with a composition greater than 50% w/w Cu.

Preferably, substantially all exposed support surfaces of the handle are antimicrobial.

Preferably, at least one LED emits blue or blue/violet wavelength light. Preferably, at least one LED emits a broad-spectrum light. Preferably, at least one LED emits with a color temperature in the region of 2000K to 10500K, and optionally said LED is a broad-spectrum wavelength LED. Preferably, the rod engages in said at least one support a tubular gasket having an internal reflective surface acting as an optical mixing chamber.

Preferably, the rod engages in said at least one support a tubular gasket having an internal reflective surface acting as an optical mixing chamber, and the rod and the gasket are configured to provide said chamber in a space between an end of the rod and a base of the gasket. Preferably, the gasket is tapered, widening in the distal direction away from the LEDs.

Preferably, at least one support comprises a removable part releasably engaging the base and the rod. Preferably, the base comprises at least one pin or socket and the removable part comprises an opposed inter-engaging socket or pin. Preferably, at least one support comprises a body with an enclosure for a substrate for the LEDs and at least part of an LED drive circuit.

Preferably, the substrate is exposed by removal of the removable part from the base.

Preferably, the substrate supports a constant current drive circuit and LED components on a single PCB assembly, and the circuit may be DC powered for high efficiency constant current operation.

We also describe an apparatus comprising a handle of any example described herein. The apparatus may comprise a cabinet door, a trolley, a hand rail, or a door other than of a cabinet.

### Additional Statements

We describe a handle for a cabinet or other item, the handle comprising a translucent tube or rod with a photocatalytic antimicrobial external coating, and at least one support each engaging an end of the rod and being adapted to be affixed to a cabinet door, and at least one support supporting an array of at least one LED for emission of light into the rod.

Preferably, at least one LED emits blue or blue/violet wavelength light. Preferably, at least one LED emits a broad-spectrum light. Preferably, at least one LED emits with a color temperature in the region of 2000K to 10500K. Preferably, said LED is a broad-spectrum wavelength LED.

Preferably, the coating includes TiO2. Preferably, the rod (2) comprises borosilicate glass material coated with an antimicrobial TiO2 coating. Preferably, the TiO2 coating is a nano TiO2 polymer, withTiO2 nanoparticles, with particle sizes of average diameter between 1nm and 15nm. Preferably, the antimicrobial coating has a thickness in the range of 50nm of 120nm.

Preferably, the rod includes a mirror for reflection of emitted light within the rod. Preferably, the mirror forms an end wall of the rod, opposed to the LEDs.

Preferably, the LEDs and the rod, and a mirror if present, are adapted to cause emitted light to mix and propagate through the rod with total internal reflection.

Preferably, the rod engages in the support a tubular gasket having an internal reflective surface acting as an optical mixing chamber. Preferably, the gasket is tapered, widening in the distal direction away from the LEDs. Preferably, the at least one support (3, 4) comprises a base (10) for fixing to a cabinet door or other apparatus, and a removable part releasably engaging the base.

Preferably, the base comprises at least one pin or socket and the removable part comprises an opposed inter-engaging socket or pin. Preferably, at least one support comprises a body with an enclosure for a substrate 20 for the LEDs and at least part of an LED drive circuit. Preferably, the substrate is exposed by removal of the removable part (11) from the base (10).

Preferably, the substrate supports a constant current drive circuit and LED components on a single PCB assembly. Preferably, the circuit is DC powered with for example 6V to 48V input working range, for high efficiency constant current operation.

Preferably, at least one support includes an antimicrobial alloy. Preferably, the alloy includes Cu, preferably with a composition greater than 50% w/w Cu. Preferably, substantially all exposed support surfaces are antimicrobial. Preferably, the handle comprises at least two supports.

Preferably, there are two opposed supports, one at each end of the rod.

We also describe an apparatus including a handle of any example described herein, the apparatus being for example s cabinet, a trolley, a hand rail such as a banister, or a door for other than a cabinet. In one example the apparatus is a retail store cabinet, and the handle is affixed to a door of the cabinet.

### Detailed Description of the Invention

The invention will be more clearly understood from the following description of some embodiments thereof, given by way of example only with reference to the accompanying drawings in which:

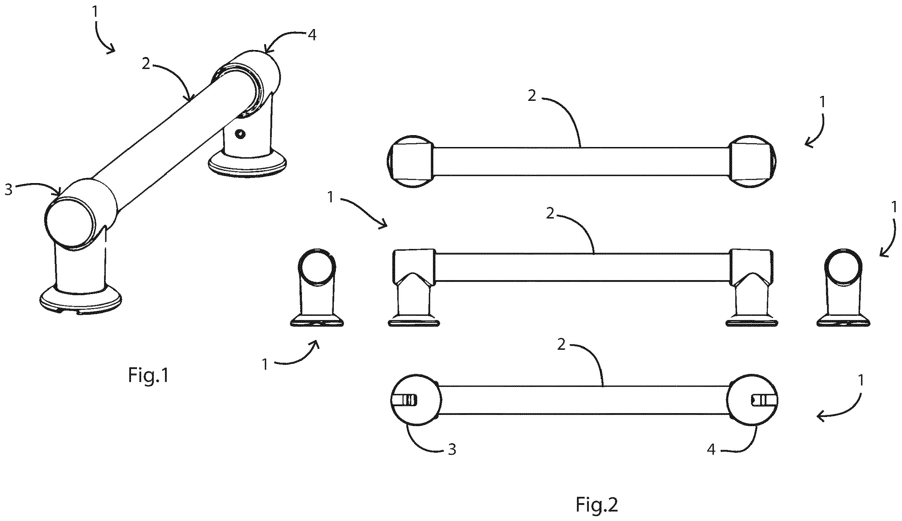

Fig. 1 is a perspective view of a handle for a refrigerated cabinet, and Fig. 2 is a set of elevation, top plan, bottom plan, and both side views of the handle;

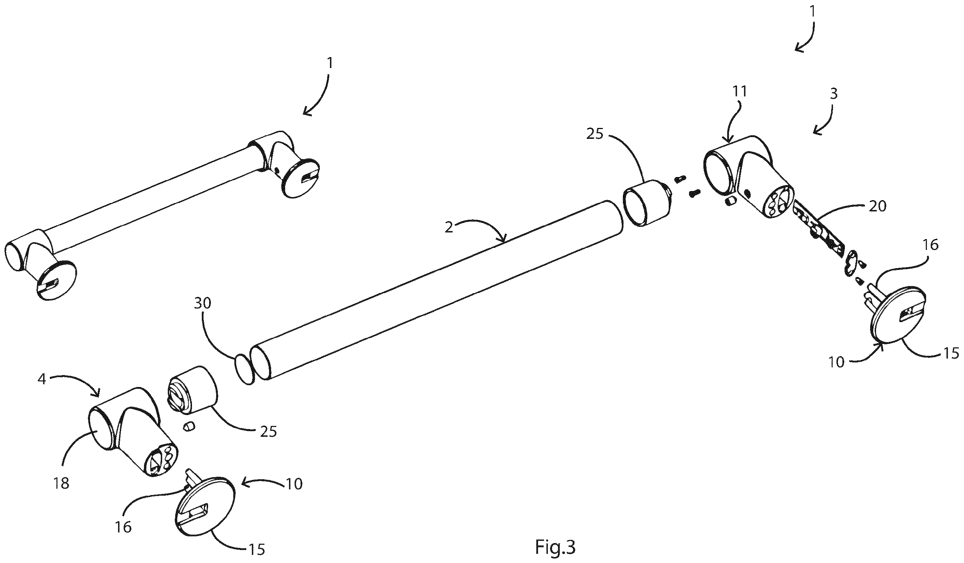

Fig. 3 is an exploded view of the handle;
Figs. 4(a) and 4(b) are a pair of opposed perspective exploded views of a support of the handle, and Figs. 5(a) and (b) are equivalent views of the other support;

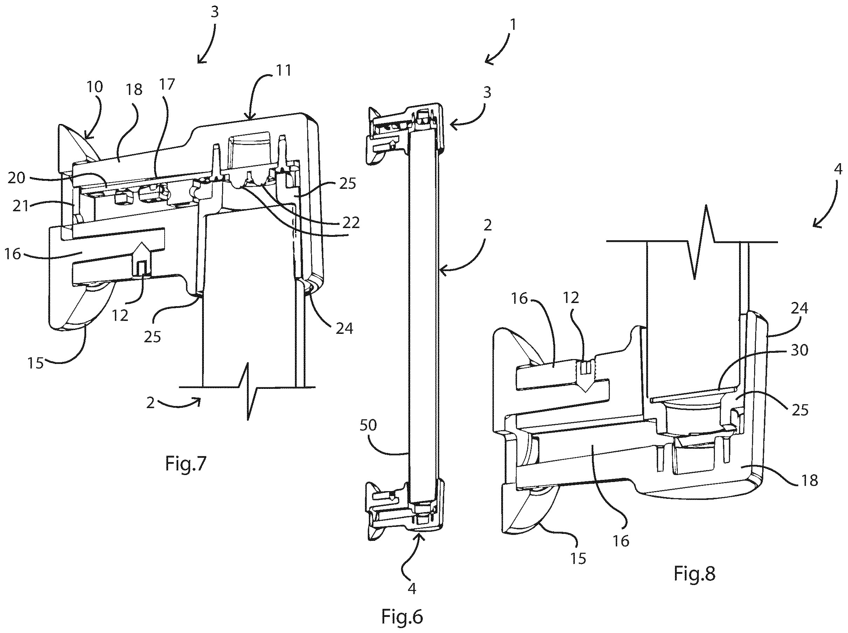

Fig. 6 is a cut-away view of the full handle;
Fig. 7 is a cut-away view of a support, showing internal components in more detail, and Fig. 8 is a similar view of the other support; and
Fig. 9 is a spectral power distribution plot for LEDs of the handle.

### Description of the Embodiments

Referring to the drawings there is illustrated a handle 1 for a cabinet such as a refrigerated cabinet in a retail store. The handle 1 comprises a hand-grasp rod 2 and end supports 3 and 4 for supporting the rod and engaging a cabinet door. The handle is suited to doors of either the hinged or sliding types. As is described in detail below the rod may be easily removed for replacement or repair, and the handle is modular and robust in nature. This allows it to be used in a high-usage situation in a retail environment, where it is particularly important to prevent contamination of users' hands.

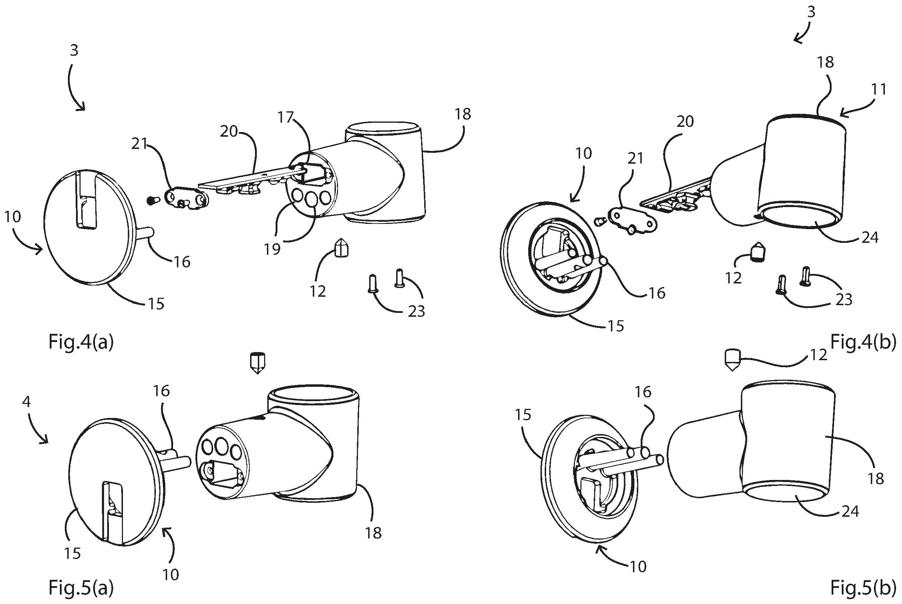

The support 3 comprises a base 10 for engaging a cabinet door, and a removable part 11 for engaging the rod 2. The base 10 comprises a copper (Cu) foot 15 and an integrally cast set of three pins 16 extending from the foot. The removable part 11 comprises a cast (or sintered in other examples) copper (Cu) body 18 which is configured with sockets 19 to receive the pins 16, and a side opening to receive a grub screw 12 to contact a pin 16 to removably retain the part 11 in engagement with the base 10. The body 18 has a socket enclosure 17 to receive an elongate circuit board 20. A blanking cover 21 is provided for fastening the board 20 in place in the enclosure 17 formed by the body 18. This is best illustrated in Figs. 4 and 5.

The board 20 supports a pair of juxtaposed LEDs. The Cu body 18 forms a socket 24 to receive a tubular gasket 25 for the glass rod 2, to hold it in registry with the LEDs 22.

The construction of the handle 1 enables a PCB to have a constant current drive circuit and LED components on a single PCB assembly for ease of assembly and increased reliability. The circuit is DC powered with for example 6V to 48V input working range, for high efficiency constant current operation. The gasket 25 is held in place within the socket 24 by fastening screws 23. The internal surface of the gasket 25 acts as a tapered optical mixing chamber, widening from the LEDs distally towards the opposite end of the rod 2, on the end face of which there is a mirror.

At the opposite end the support 4 comprises the base 10 and the body 18, and like parts are indicated by the same reference numerals. Although the support 4 includes the body 18 it does not support a circuit board, nor LEDs. It is convenient to use the same parts for interchangeability and convenience, and they work in the same way for engaging the base 10 to allow easy changing of the rod 2. The body 18 of the support 4 also has the socket 24 to receive another gasket 25, but in addition the gasket 25 at this end supports a disc-shaped mirror 30 forming an end face of the solid rod. This contributes to total internal reflection within the rod of light emitted by the LEDs. Such light propagation is particularly effective for incidence on the coating 50.

The exposed parts of the supports 3 and 4, including the base feet 15 and the bodies 18, are of Cu alloy of the type for antimicrobial activity. The antimicrobial copper alloy has a composition greater than 50% Copper (Cu). All of these exposed metal elements on the handle deliver continuous and ongoing antibacterial action, which is effective in killing greater than 99% of bacteria within 2 hours. Such antimicrobial action may be achieved by a material other than metal, such as a polymer with an antimicrobial coating.

The rod 2 is a borosilicate glass rod coated with an antimicrobial TiO2 coating 50 with a thickness preferably in the range of 50nm to 120nm. The rod 2 is polished on both ends. Mixing of the spectral energies from both LEDs is performed in both the mixing chamber (within the gasket 25 volume at the emission end) and internally within the glass rod 2 along its length. The gaskets 25 are in this example of a white silicone material, with smooth polished internal surface. In this case the mirror 30 comprises a high reflectance polyester film.

The photocatalyst coating 50 on the external surface of the rod 2 can continuously decompose pollutants with exposure to light from the LEDs 22. The TiO2 transparent (or, in other examples, semi-transparent) coating mix is applied using a dip or spay method to the glass. The TiO2 coating is a nano TiO2 polymer, which contains TiO2 nanoparticles, with particle sizes of average diameter between 1nm to 15nm. The size of the TiO2 nanoparticles is proportional to the photocatalytic efficiency of the process and is matched to the multi-peak blue-violet and broad visible spectrum. Titanium dioxide (TiO2) is safe, nontoxic and harmless. It is widely used in a variety of products such as cosmetics, including lip balms and sunscreens. The Scientific Committee on Consumer Safety (SCCS) considers nano-TiO2 as a non-sensitizer and as mild- or non-irritant to skin. TiO2 does not penetrate through healthy skin and poses no local or systemic risk to human health from skin exposure.

Interest in nano-TiO2 started with the discovery of its catalytic properties induced by UV radiation, in the beginning of the 1970s. The self-cleaning effect of TiO2 under ultraviolet (UV) irradiation due to the photoinduced hydrophilic properties of TiO2 is well known. Clear and unified explanations have been made regarding the mechanisms of the TiO2 photocatalysis process. A series of reactions have been induced by the photogenerated e- (electrons) and h+ (holes) from the photoexcited catalyst in the system. Reactive oxygen species are generated on the surface of the titanium dioxide nanoparticles. Superoxide anion radicals O2, hydrogen peroxide H2O2 and hydroxyl radicals (OH) are produced as a result of these different photoreactions and then facilitated the decomposition of the organic substances. These reactive oxygen species not only inactivate but also oxidize viruses, bacteria, organic pollutants, yeasts and moulds, converting them to carbon dioxide (CO2) and water (H2O). This effect leads to the antimicrobial property of nano-TiO2 and its ability to self-clean, along with eradicating pathogenic microorganisms on the surface of the handle.

The LEDs 22 are of different wavelengths, and have a combined power consumption of circa 2W. One LED is white and the other is blue. This achieves activation of the antimicrobial activity without need for dangerous UV light. The gasket 25 at the LED end and the rod 2 cause the emitted light to mix and propagate through the rod with total internal reflection.

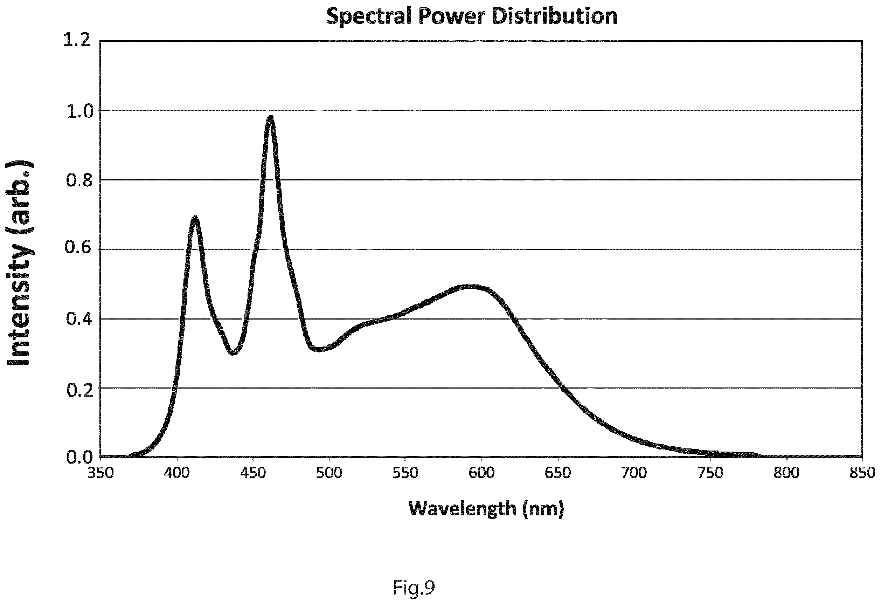

One LED 22 is a BLUE/Violet LED and the other is a broad-spectrum white LED in the region of 2000K to 10500K. This creates a targeted multi-peak in the blue-violet wavelengths and additionally has a broad visible spectrum that activates the titanium dioxide coating on the glass. A sample spectrum is shown in Fig. 9. The importance of this spectrum is two-fold, firstly it is optimized to promote the highest efficiency of the TiO2 photocatalyst process. Secondly it should be noted that the International Electrotechnical Commission (IEC) has established objective indicators to measure and evaluate the impact of LED spectrums on the human body. This is described in their published photo-biological safety standards in IEC 62471. This standard sets specific exposure limits and measurement techniques to asses and manage the photobiological hazards of light sources emitting light in the wavelength range of 200nm to 3000nm. The spectrum combination as well as the difference in intensities is designed to ensure no risk of either retinal thermal hazard or blue light hazard (photoretinitis).

The invention is not limited to the embodiments described but may be varied in construction and detail. The handle described is suited to use on a cabinet. However, a handle of the invention may be suited to any of a wide variety of uses where there is frequent human contact. Examples are doors other than of cabinets, handrails such as for a staircase, or a shopping trolley handle. Also, it is envisaged that a handle of the invention may be fixed at only one end and open at the other end. In this case the support may need to be larger, and its socket to receive the rod may be deeper, and the glass rod itself may be of a more robust material such as a polymer.

It is envisaged that in other examples the support(s) are of a different material, for example a polymer or a metal which is not primarily Cu-based.

The LED array has at least one LED, and there may be any desired number to provide the required light intensity and wavelength(s). The LED wavelengths may be different. For example, there may be only a single white LED with a single colour temperature (CCT), or alternatively a single Blue LED, however white and blue is preferred. Alternatively, there may be multiple LEDS with different but close peaks, such as two white LEDs of differing CCT, thus creating two (overlapping) peaks in the BLUE and also broad spectrum. In general, it is preferred that there is a visible spectrum without UV and so the handle poses no risk of photo-biological safety. Also, in other examples, the rod may be hollow or have an internal compartment in which LEDs are mounted internally. However, it is preferred that the rod be solid with light entering at one or both ends and reflecting internally.

## Claims

1. A handle (1) comprising:
a translucent hand-grip rod (2) with a photocatalytic antimicrobial external coating (50),
at least one support (3, 4) engaging an end of the rod and comprising a base (10, 15) adapted to be affixed to an apparatus, and
an array of at least one LED (22) for emission of light to activate the coating.

2. A handle as claimed in claim 1, wherein the handle comprises at least two said supports (3, 4), each engaging an end of the rod.

3. A handle as claimed in either of claims 1 or 2, wherein the LED array (17, 22) is mounted in at least one said support (3) for emission of light into an end of the rod (2), and wherein the rod is configured for total internal reflection.

4. A handle as claimed in claim 3, wherein the rod has a mirror (30) across an end of the rod opposite to the end through which emitted light enters.

5. A handle as claimed in any preceding claim, wherein the rod (2) comprises borosilicate glass material.

6. A handle as claimed in any preceding claim, wherein the coating includes TiO2.

7. A handle as claimed in claim 7, wherein the TiO2 coating comprises a nano TiO2 polymer, with TiO2 nanoparticles, with particle sizes of average diameter between 1nm and 15nm, and the rod antimicrobial coating (50) has a thickness in the range of 50nm to 120nm.

8. A handle as claimed in any preceding claim, wherein at least one support includes an antimicrobial exposed surface, and optionally the support comprises an alloy including Cu with a composition greater than 50% w/w Cu.

9. A handle as claimed in any preceding claim, wherein at least one LED emits blue or blue/violet wavelength light.

10. A handle as claimed in any preceding claim, wherein at least one LED emits with a color temperature in the region of 2000K to 10500K, and optionally said LED is a broad-spectrum wavelength LED.

11. A handle as claimed in any preceding claim, wherein the rod engages in said at least one support a tubular gasket (25) having an internal reflective surface and the rod and the gasket are configured to provide said chamber in a space between an end of the rod and a base of the gasket, and optionally the gasket (25) is tapered, widening in the distal direction away from the LEDs.

12. A handle as claimed in any preceding claim, wherein at least one support comprises a removable part (11, 18) releasably engaging the base (10, 15) and the rod (2), and the base comprises at least one pin (16) or socket and the removable part comprises an opposed inter-engaging socket (19) or pin.

13. A handle as claimed in any preceding claim, wherein at least one support comprises a body (18) with an enclosure (17) for a substrate (20) for the LEDs (22) and at least part of an LED drive circuit, and wherein the substrate is exposed by removal of the removable part (11) from the base (10).

14. An apparatus comprising a handle of any preceding claim.

15. An apparatus of claim 14, wherein the apparatus comprises a cabinet having a door, and the handle is affixed to the door, and optionally the handle has a support engaging the rod at each end of the rod and both said supports are affixed to the door.
